# EUROPEAN PATENT APPLICATION

(11) **EP 3 034 586 A1**
(43) Date of publication of application: **22.06.2016**
(21) Application number: 14835975.5
(22) Date of filing: 14.08.2014
(51) Int. Cl.: C10M 133/24, C10M 129/10, C10M 129/70, C10M 133/04, C10M 133/16, C10M 135/00, C10M 137/00, C10N 30/06, C10N 40/02, C10N 40/04, C10N 40/25

(54) **LUBRICANT OIL ADDITIVE AND LUBRICANT OIL COMPOSITION**

(30) Priority: 16.08.2013 JP 2013169099
(71) Applicant: JX Nippon Oil & Energy Corporation, Chiyoda-ku Tokyo 100-8162 (JP)
(72) Inventor: HOSHINO, Koji, Tokyo 100-8162 (JP); UENO, Ryuichi, Tokyo 100-8162 (JP); HASEGAWA, Shinji, Tokyo 100-8162 (JP)
(74) Representative: Hoffmann Eitle
(86) International application number: PCT/JP2014/071447
(87) International publication number: WO 2015/022990

(57) **Abstract**

A lubricant additive including a compound represented by a general formula (1): 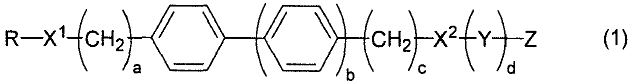 (In the general formula (1), R is a hydrocarbon-containing group having a carbon number of no less than 1; X¹ is a single bond, -CONH- group, -NHCO- group, -NH- group, or a linking group having a carbon number of 0 to 1 and having no less than one heteroatom and not having a hydrogen atom bonded to the heteroatom; X² is a single bond, or a linking group having a carbon number of 0 to 1 and having no less than one heteroatom and not having a hydrogen atom bonded to the heteroatom; Z is -CN group, -CO₂CH₃ group, or a group having a carbon number of 0 to 6 and having no less than one heteroatom and a hydrogen atom bonded to the heteroatom; a is 0 or 1; b is 1 or 2; c is 0 or 1; d is an integer of 0 to 2; and Y is -CH₂- group or -OCH₂CH₂- group where d is no less than 1.).

## Description

### Technical Field

The present invention relates to a lubricant additive and a lubricant oil composition.

### Background Art

Lubricant oils are used for internal combustion engines, automatic transmissions, bearings, and the like, in order to make their functions smooth. Generally, various additives are incorporated in lubricant oils to make the lubricant oils have required performances. For example, lubricant additives such as anti-wear agents, friction modifiers, metallic detergents, ashless dispersants, and antioxidants are incorporated in common engine oils.

Among lubricant additives, additives having a function of reducing friction resistance (friction modifier: hereinafter may be referred to as "FM") are important components to reduce the energy loss due to friction. FMs generally used can be classified into organic molybdenum FMs including molybdenum and oiliness agent FMs which reduce friction by improving oiliness.

As an organic molybdenum FM, MoDTC (molybdenum dithiocarbamate) and MoDTP (molybdenum dithiophosphate) are widely known (see Patent Literature 1 for example). These organic molybdenum FMs have good friction reducing effects at initial stages of use. However, they have limitations in sustaining the friction reducing effects for long time at good conditions. In addition, organic molybdenum FMs, which include ash, make it difficult to reuse used lubricating oils, and if used for lubrication of internal combustion engines, it may negatively affect the exhaust gas purifying devices. Therefore, there is a demand for reducing the additive amount of organic molybdenum FMs.

On the other hand, with oiliness agent FMs, there is a possibility of overcoming the above problems of organic molybdenum FMs. Therefore, oiliness agent FMs are becoming increasingly important, in view of environmental responsibility (see Patent Literatures 2 and 3 for example).

### Citation List

### Patent Literature

Patent Literature 1: JP 2013-133453 A
Patent Literature 2: JP 2009-235252 A
Patent Literature 3: JP 2006-257383 A

### Summary of Invention

### Technical Problem

The present invention provides a lubricant additive including a new ashless oiliness agent friction modifier. The present invention also provides a lubricant oil composition including the oiliness agent friction modifier.

### Solution to Problem

A first aspect of the present invention is a lubricant additive including a compound represented by a general formula (1): 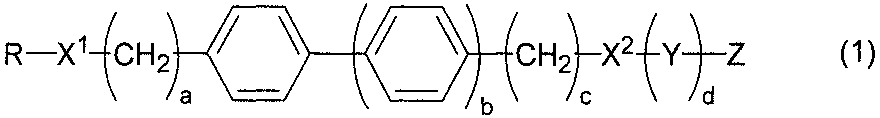 (In the general formula (1), R is a hydrocarbon-containing group having a carbon number of no less than 1; X¹ is a single bond, -CONH- group, -NHCO- group, -NH- group, or a linking group having a carbon number of 0 to 1 and having no less than one heteroatom and not having a hydrogen atom bonded to the heteroatom, wherein the heteroatom is selected from a group consisting of: oxygen, nitrogen, and sulfur; X² is a single bond, or a linking group having a carbon number of 0 to 1 and having no less than one heteroatom and not having a hydrogen atom bonded to the heteroatom, wherein the heteroatom is selected from a group consisting of: oxygen, nitrogen, and sulfur; Z is -CN group, -CO₂CH₃ group, or a group having a carbon number of 0 to 6 and having no less than one heteroatom and a hydrogen atom bonded to the heteroatom, wherein the heteroatom is selected from a group consisting of: oxygen, nitrogen, sulfur, and phosphorus; a is 0 or 1; b is 1 or 2; c is 0 or 1; d is an integer of 0 to 2; and Y is -CH₂- group or -OCH₂CH₂- group where d is no less than 1.).

A second aspect of the present invention is a lubricant oil composition including (A) a lubricating base oil; and (B) a compound represented by the above general formula (1).

The lubricant oil composition according to the second aspect of the present invention may include one or more additive selected from a group consisting of: an ashless dispersant, an antioxidant, a friction modifier other than the compound represented by the general formula (1), an anti-wear agent, an extreme pressure agent, a metallic detergent, a viscosity index improver, a pour point depressant, a corrosion inhibitor, an anti-rust agent, a metal deactivator, a demulsifier, a deforming agent, and a coloring agent, in addition to (A) the lubricating base oil and (B) the compound represented by the above general formula (1).

### Advantageous Effects of Invention

The compound represented by the above general formula (1) functions as an ashless oiliness agent friction modifier. The lubricant additive according to the first aspect of the present invention can provide a good friction reducing effect under conditions of boundary lubrication.

The lubricant oil composition according to the second aspect of the present invention is a lubricant oil composition including the ashless oiliness agent friction modifier, and can provide a good friction reducing effect under conditions of boundary lubrication.

### Brief Description of Drawings

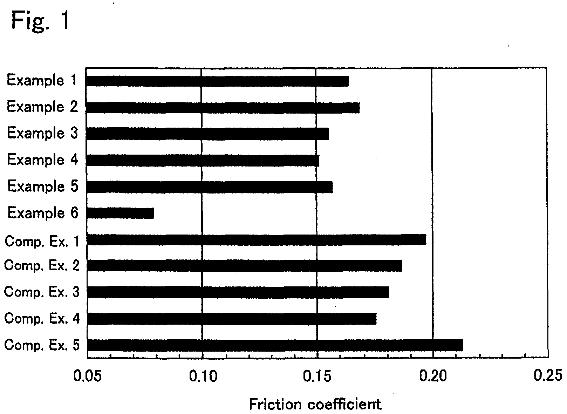

Fig. 1 is a graph showing the evaluation results of the friction characteristic of lubricant oil compositions according to Examples and Comparative Examples.

### Description of Embodiments

Hereinafter the present invention will be described in detail. It is noted that, unless otherwise mentioned, "A to B" regarding numerical values A and B means "no less than A and no more than B". In a case where the unit of the numerical value A is omitted, the unit given to the numerical value B is applied as the unit of the numerical value A.

### <1. Lubricant additive>

The lubricant additive according to the first aspect of the present invention includes one or more of the compound represented by the following general formula (1).

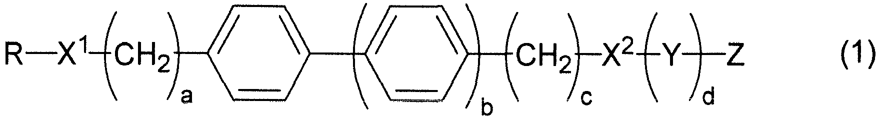

In the general formula (1), a is 0 or 1, b is 1 or 2, c is 0 or 1. The compound represented by the general formula (1) has a biphenyl moiety or a terphenyl moiety.

In the general formula (1), R is a hydrocarbon-containing group having a carbon number of no less than 1, typically a hydrocarbon group having a carbon number of no less than 1. However, as long as R has a hydrocarbon group, R may have no less than one heteroatom selected from a group consisting of oxygen, nitrogen, and sulfur, in its molecular structure.

Examples of the hydrocarbon group include: alkyl groups (which may have a ring structure); alkenyl groups (whose double bond(s) may be in any position, and which may have a ring structure); aryl groups (which may have an alkyl group or an alkenyl group); arylalkyl groups, and arylalkenyl groups.

Examples of the alkyl groups include various linear or branched alkyl groups. Examples of the ring structure the alkyl groups may have include cycloalkyl groups having a carbon number of 5 to 7, such as cyclopentyl group, cyclohexyl group, and cycloheptyl group. In a case where a chain hydrocarbon group is substituted on the ring structure, substitution on the ring structure may be in any position.

Examples of the alkenyl groups include various linear or branched alkenyl groups. Examples of the ring structure the alkenyl groups may have include the above-described cycloalkyl groups, and cycloalkenyl groups having a carbon number of 5 to 7, such as cyclopentenyl group, cyclohexenyl group, and cycloheptenyl group. In a case where a chain hydrocarbon group is substituted on the ring structure, substitution on the ring structure may be in any position.

Examples of the aryl groups include phenyl group and naphthyl group. In the alkylaryl groups, alkenylaryl groups, arylalkyl groups, and arylalkenyl groups, substitution on aromatic ring may be in any position.

The carbon number of R is no less than 1, normally no more than 40, typically no more than 30. The carbon number of R is preferably no less than 6, more preferably no less than 13, especially preferably no less than 15. The hydrocarbon group of R is preferably an aliphatic hydrocarbon group, more preferably a linear aliphatic hydrocarbon group.

In the general formula (1), X¹ is a single bond, -CONH- group, -NHCO- group, -NH- group, or a linking group having a carbon number of 0 to 1 and having no less than one heteroatom and not having a hydrogen atom bonded to the heteroatom. Regarding X¹, the "heteroatom" is selected from oxygen, nitrogen, and sulfur, and typically oxygen or nitrogen. Regarding X¹, the "linking group having a carbon number of 0 to 1 and having no less than one heteroatom and not having a hydrogen atom bonded to the heteroatom" may be selected from -O- group, -S- group, -S(O)- group, -S(O)₂- group, -S(O)₂O- group, -OS(O)₂- group, -NCH₃- group, -C(O)- group, -OC(O)- group, -C(O)O- group, and the like for example.

In the general formula (1), X² is a single bond, or a linking group having a carbon number of 0 to 1 and having no less than one heteroatom and not having a hydrogen atom bonded to the heteroatom. Regarding X², the "heteroatom" is selected from oxygen, nitrogen, and sulfur, and typically oxygen or nitrogen. Regarding X², the "linking group having a carbon number of 0 to 1 and having no less than one heteroatom and not having a hydrogen atom bonded to the heteroatom" may be selected from -O- group, -S- group, -S(O)-group, -S(O)₂ group, -S(O)₂O- group, -OS(O)₂- group, -NCH₃- group, -C(O)- group, -OC(O)-group, -C(O)O- group, and the like.

In the general formula (1), Z is -CN group, -CO₂CH₃ group, or a group having a carbon number of 0 to 6 and having no less than one heteroatom and a hydrogen atom bonded to the heteroatom. Regarding Z, the "heteroatom" is selected from oxygen, nitrogen, sulfur, and phosphorus, and typically oxygen or nitrogen. In a case where the "group having a carbon number of 0 to 6 and having no less than one heteroatom and a hydrogen atom bonded to the heteroatom" has no less than two heteroatoms, it may have no less than one heteroatom to which a hydrogen atom is not bonded. Regarding Z, the "group having a carbon number of 0 to 6 and having no less than one heteroatom and a hydrogen atom bonded to the heteroatom" may be selected from -OH group, -CO₂H group, -NH₂ group, -NHR' group (R' is a hydrocarbyl group), -NHCOR' group (R' is a hydrocarbyl group), -CONH₂ group, -CONHR' group (R' is a hydrocarbyl group), -SH group, -SOH group, -S(O)H group, - S(O)OH group, -S(O)₂H group, -S(O)₂OH group, -OS(O)₂H group, -P(OH)₂ group, -P(OR')OH group (R' is a hydrocarbyl group), -PH(O)OH group, -P(O)(OH)R' group (R' is a hydrocarbyl group), -OPH(OH) group, -OP(OH)R' group (R' is a hydrocarbyl group), -P(O)(OH)₂ group, -P(O)(OR')OH group, (R' is a hydrocarbyl group), -OP(OH)₂ group, -OP(OR')OH group (R' is a hydrocarbyl group), -OPH(O)OH group, -OPH(O)OR' group (R' is a hydrocarbyl group), -OP(O)(OH)R' group (R' is a hydrocarbyl group), -OP(O)(OH)₂ group, -OP(O)(OR')OH group (R' is a hydrocarbyl group), and the like. Regarding Z, the carbon number of the "group having a carbon number of 0 to 6 and having no less than one heteroatom and a hydrogen atom bonded to the heteroatom" is preferably 0 to 5, and more preferably 0 to 3.

In the general formula (1), d is an integer of 0 to 2, preferably 0 or 1, more preferably 0. Y is -CH₂- group or -OCH₂CH₂- group where d is no less than 1, and typically -CH₂- group. It is preferable that the compound represented by the general formula (1) does not have an -O-O- bond.

The content of the compound represented by the above general formula (1) in the lubricant additive of the present invention is not particularly limited. For example, the content may be an amount with which a normal or preferable content of (B) component in the lubricant oil composition of the present invention described later is realized.

### <2. Lubricant oil composition>

The lubricant oil composition according to the second aspect of the present invention includes (A) a lubricating base oil and one or more of (B) compound represented by the general formula (1) (hereinafter may be referred to as "biphenyl-based ashless friction modifier")

### ((A) Lubricating base oil)

The lubricating base oil in the lubricant oil composition of the present invention is not particularly limited and mineral base oils and synthetic base oils used for general lubricant oils may be used.

Specific examples of the mineral base oils include: a product made by purifying a lubricating oil fraction obtained by vacuum distillation of a residue of atmospheric distillation of a crude oil, by one or more treatments of solvent deasphalting, solvent extraction, hydrogenolysis, solvent dewaxing, hydrorefining, and the like; an wax-isomerized mineral oil; a lubricating oil base oil produced by a method of isomerizing a GTL WAX (Gas To Liquid wax) produced for example by Fisher-Tropsh process, and the like.

Examples of the synthetic base oils include: a poly α-olefin such as 1-octene oligomer and 1-decene oligomer, and hydrogenation product thereof; isobutene oligomer and hydrogenation product thereof; paraffin; alkylbenzene; alkylnaphtalene; diesters (e.g. ditridecyl glutarate, di-2-ethylhexyl adipate, diisodecyl adipate, ditridecyl adipate, and di-2-ethylhexyl sebacate); polyol esters (e.g. trimetylolpropane caprylate, trimetylolpropane pelargonate, pentaerythritol-2-ethylhexanoate, and pentaerythritol pelargonate); polyoxyalkyleneglycol; dialkyldiphenyl ether; and polyphenyl ether. In addition, aromatic synthetic oils such as alkylnaphthalene, alkylbenzene, and aromatic ester, and mixture thereof may be given as examples.

In the lubricant oil composition of the present invention, mineral base oils, synthetic base oils, or any mixture of two or more lubricant oils selected therefrom and the like may be used as the lubricating base oil. For example, one or more mineral base oils, one or more synthetic base oils, a mixture oil of one or more mineral oils and one or more synthetic base oils, and the like may be given.

The kinematic viscosity, NOACK evaporation loss, and viscosity index of the lubricating base oil in the lubricant oil composition of the present invention may be selected depending on the purpose of use of the lubricant oil composition. For example, in a case where the lubricant oil composition is for internal combustion engines, the kinematic viscosity of the lubricating base oil at 100°C may be 3.0 to 16.3 mm²/s. For example, in a case where the lubricant oil composition is for transmissions, the kinematic viscosity of the lubricating base oil at 100°C may be 3.5 to 25.0 mm²/s.

### ((B) Biphenyl-based ashless friction modifier)

The details of the compound (B) represented by the above formula (1) are as already described regarding the lubricant oil composition according to the first aspect of the present invention. The content of (B) component is not particularly limited, and for example may be 0.05 to 10 weight% to the total amount of the composition, and preferably no more than 5 weight%. The preferable range of the content depends on the purpose of use of the lubricant oil composition, and for example preferably no less than 0.1 weight% and preferably no more than 5 weight%.

### (Other additives)

The lubricant oil composition according to the second aspect of the present invention may further include, in addition to the above-described (A) lubricating base oil and (B) biphenyl-based ashless friction modifier, one or more additive selected from a group consisting of (C) an ashless dispersant, (D) an antioxidant, (E) a friction modifier other than the compound represented by the general formula (1), (F) an anti-wear agent or an extreme pressure agent, (G) a metallic detergent, (H) a viscosity index improver or a pour point depressant, (I) a corrosion inhibitor, (J) an anti-rust agent, (K) a metal deactivator, (L) a demulsifier, (M) a deforming agent, and (N) a coloring agent. It is noted that an additive package may be made by including one or more additive selected from the group consisting of (C) to (N), in the lubricant additive according to the first aspect of the present invention.

As (C) an ashless dispersant, a known ashless dispersant such as a succinimide-based ashless dispersant may be used. In a case where an ashless dispersant is included in the lubricant additive of the present invention, the content thereof is, on the basis of the total amount of the lubricant oil composition, that is, considering the total amount of the lubricant oil composition as 100 weight%, normally no less than 0.01 weight% and preferably no less than 0.1 weight%; and normally no more than 20 weight%, and preferably no more than 10 weight%.

As (D) an antioxidant, a known antioxidant such as a phenol based antioxidant and amine based antioxidant may be used (excepting the compound represented by the above general formula (1)). In a case where an antioxidant is included in the lubricant oil composition of the present invention, the content thereof is, on the basis of the total amount of the lubricant oil composition, normally no more than 5.0 weight% and preferably no more than 3.0 weight%; and preferably no less than 0.1 weight%, and more preferably no less than 0.5 weight%.

As (E) a friction modifier other than the compound represented by the above general formula (1), a known friction modifier may be used. Examples thereof include oiliness agent friction modifiers such as fatty acid esters, and organic molybdenum friction modifiers. In a case where these friction modifiers are included in the lubricating oil composition of the present invention, the content thereof is, on the basis of the total amount of the lubricant oil composition, normally no less than 0.05 weight% and no more than 5 weight%.

As (F) an anti-wear agent or an extreme pressure agent, a known anti-wear agent or extreme pressure agent may be used. Examples thereof include phosphorus compounds such as zinc dithiophosphate, sulfur-containing compounds such as disulfides and sulfide oils (excepting the compound represented by the above general formula (1)). In a case where these anti-wear agents are included in the lubricant oil composition of the present invention, the content thereof is, on the basis of the total amount of the lubricant oil composition, normally no less than 0.05 weight% and no more than 5 weight%.

As (G) a metallic detergent, a known metallic detergent may be used. Examples thereof include alkali metal sulfonates, alkaline earth metal sulfonates, alkali metal phenates, alkaline earth metal phenate, alkali metal salicylates, alkaline earth metal salicylates, and mixture thereof. These metallic detergents may be overbased. In a case where a metallic detergent is included in the lubricant oil composition of the present invention, the content thereof is not particularly limited. In a case where the lubricant oil composition is for automatic transmissions or continuously variable transmissions, the content thereof is, as a metal content on the basis of the total amount of the lubricant oil composition, normally no less than 0.01 weight% and no more than 5 weight%. In a case where the lubricant oil composition is for internal combustion engines, the content thereof is, as a metal content on the basis of the total amount of the lubricant oil composition, normally no less than 0.01 weight% and no more than 1.0 weight%.

As (H) a viscosity index improver or a pour point depressant, a known viscosity index improver or pour point depressant may be used. Examples of the viscosity index improver include: a so-called non-dispersant type viscosity index improver such as a polymer or copolymer of monomers of one or two or more selected from various methacrylic acid esters, and hydrogenated products thereof; a so-called dispersant type viscosity index improver obtained by copolymerizing various methacrylic acid esters including a nitrogen compound; a non-dispersant type or dispersant type ethylene-α-olefin copolymer and hydrogenated product thereof; polyisobutylene and a hydrogenated product thereof; a hydrogenated product of styrene-diene copolymer; styrene-maleic anhydride ester copolymer; polyalkylstyrene, and the like. In a case where these viscosity index improvers are included in the lubricant oil composition of the present invention, the content thereof is, on the basis of the total amount of the lubricant oil composition, normally no less than 0.1 weight% and no more than 20 weight%. Examples of the pour point depressant include polymethacrylate-based polymers. In a case where a pour point depressant is included in the lubricant oil composition of the present invention, the content thereof is, on the basis of the total amount of the lubricant oil composition, normally no less than 0.01 weight% and no more than 1 weight%.

As (I) a corrosion inhibitor, a known corrosion inhibitor such as a benzotriazole-based compound, tolyltriazole-based compound, thiadiazole-based compound, and imidazole-based compound may be used. In a case where these corrosion inhibitors are included in the lubricant oil composition of the present invention, the content thereof is, on the basis of the total amount of the lubricant oil composition, normally no less than 0.005 weight% and no more than 5 weight%.

As (J) an anti-rust agent, a known anti-rust agent such as a petroleum sulfonate, alkylbenzene sulfonate, dinonylnaphthalene sulfonate, alkenylsuccinic acid ester, and polyhydric alcohol ester may be used. In a case where these anti-rust agents are included in the lubricant oil composition of the present invention, the content thereof is, on the basis of the total amount of the lubricant oil composition, normally no less than 0.005 weight% and no more than 5 weight%.

As (K) a metal deactivator, a known metal deactivator such as imidazoline, pyrimidine derivative, alkylthiadiazole, mercaptbenzothiazole, benzotriazole and derivatives thereof, 1,3,4-thiadiazole polysulfide, 1,3,4-thiadiazolyl-2,5-bisdialkyl dithiocarbamate, 2-(alkyldithio)benzoimidazole, and β-(o-carboxybenzylthio)propionitrile may be used for example. In a case where these metal deactivators are included in the lubricant oil composition of the present invention, the content thereof is, on the basis of the total amount of the lubricant oil composition, normally no less than 0.005 weight% and no more than 1 weight%.

As (L) a demulsifier, a known demulsifier such as polyalkylene glycol-based nonionic surfactants may be used for example. In a case where a demulsifier is included in the lubricant oil composition of the present invention, the content thereof is, on the basis of the total amount of the lubricant oil composition, normally no less than 0.005 weight% and no more than 5 weight%.

As (M) a deforming agent, a known deforming agent such as silicone, fluorosilicone, and fluoroalkylether may be used for example. In a case where these deforming agents are included in the lubricant oil composition of the present invention, the content thereof is, on the basis of the total amount of the lubricant oil composition, normally no less than 0.0005 weight% and no more than 1 weight%.

As (N) a coloring agent, a known coloring agent such as azo compound may be used.

The following embodiments of [1] to [45] may be given as examples for the embodiment of the lubricant additive and lubricant oil composition of the present invention.
[1] a lubricant additive including a compound represented by a general formula (1).

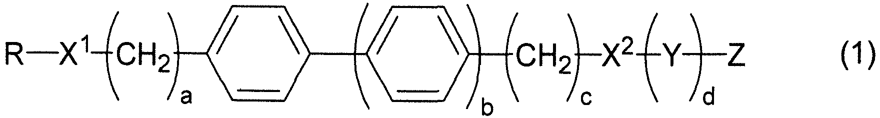

   (In the general formula (1), R is a hydrocarbon-containing group having a carbon number of no less than 1;
   X¹ is a single bond, -CONH- group, -NHCO- group, -NH- group, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
   X² is a single bond, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
   Z is -CN group, -CO₂CH₃ group, or a group having a carbon number of 0 to 6 and having no less than one heteroatom and a hydrogen atom bonded to the heteroatom, wherein the heteroatom is selected from a group consisting of: oxygen, nitrogen, sulfur, and phosphorus; a is 0 or 1; bis 1 or 2; c is 0 or 1; d is an integer of 0 to 2; and
   Y is -CH₂- group or -OCH₂CH₂- group where d is no less than 1.);
[2] an embodiment as in [1], wherein R is a hydrocarbon group having a carbon number of no less than 1;
[3] an embodiment as in [1] or [2], wherein R has a carbon number of no less than 6;
[4] an embodiment as in any one of [1] to [3], wherein R has a carbon number of no less than 13;
[5] an embodiment as in any one of [1] to [4], wherein R has a carbon number of no less than 15;
[6] an embodiment as in any one of [1] to [5], wherein R has a carbon number of no more than 40;
[7] an embodiment as in any one of [1] to [6], wherein R has a carbon number of no more than 30;
[8] an embodiment as in any one of [1] to [7], wherein R is an aliphatic hydrocarbon group;
[9] an embodiment as in any one of [1] to [8], wherein R is a linear aliphatic hydrocarbon group;
[10] an embodiment as in any one of [1] to [9], wherein X¹ is a single bond, -CONH- group, -NHCO- group, -NH- group, -O- group, -S- group, -S(O)- group, -S(O)₂- group, -S(O)₂O-group, -OS(O)₂- group, -NCH₃- group, -C(O)- group, -OC(O)- group, or -C(O)O- group;
[11] an embodiment as in any one of [1] to [10], wherein the heteroatom of X¹ is selected from a group consisting of oxygen and nitrogen;
[12] an embodiment as in any one of [1] to [11], wherein X¹ is a single bond, -CONH- group, -NHCO- group, -NH- group, -O- group, -NCH₃- group, -C(O)- group, -OC(O)- group, or -C(O)O- group;
[13] an embodiment as in any one of [1] to [12], wherein X¹ is a single bond, -O- group, or -C(O)O- group;
[14] an embodiment as in any one of [1] to [13], wherein X² is a single bond, -O- group, -S-group, -S(O)- group, -S(O)₂- group, -S(O)₂O- group, -OS(O)₂- group, -NCH₃- group, -C(O)-group, -OC(O)- group, or -C(O)O- group;
[15] an embodiment as in any one of [1] to [14], wherein the heteroatom of X² is selected from a group consisting of oxygen and nitrogen;
[16] an embodiment as in any one of [1] to [15], wherein X² is a single bond, -O- group, -NCH₃- group, -C(O)- group, -OC(O)- group, or -C(O)O- group;
[17] an embodiment as in any one of [1] to [16], wherein X² is a single bond or -C(O)O-group;
[18] an embodiment as in any one of [1] to [17], wherein Z is -CN group, -CO₂CH₃ group, -OH group, -CO₂H group, -NH₂ group, -NHR' group, -NHCOR' group, -CONH₂ group, -CONHR' group, -SH group, -SOH group, -S(O)H group, -S(O)OH group, -S(O)₂H group, -S(O)₂OH group, -OS(O)₂H group, -P(OH)₂ group, -P(OR')OH group, -PH(O)OH group, -P(O)(OH)R' group, -OPH(OH) group, -OP(OH)R' group, -P(O)(OH)₂ group, -P(O)(OR')OH group, -OP(OH)₂ group, -OP(OR')OH group, -OPH(O)OH group, -OPH(O)OR' group, -OP(O)(OH)R' group, -OP(O)(OH)₂ group, or -OP(O)(OR')OH group, R' is a hydrocarbyl group and Z has a carbon number of 0 to 6;
[19] an embodiment as in any one of [1] to [18], wherein the heteroatom of Z is selected from a group consisting of oxygen and nitrogen;
[20] an embodiment as in any one of [1] to [19], wherein Z is -CN group, -CO₂CH₃ group, -OH group, -CO₂H group, -NH₂ group, -NHR' group, -NHCOR' group, -CONH₂ group, or -CONHR' group, and R' is a hydrocarbyl group;
[21] an embodiment as in any one of [1] to [20], wherein Z has a carbon number of 0 to 5;
[22] an embodiment as in any one of [1] to [21], wherein Z has a carbon number of 0 to 3;
[23] an embodiment as in any one of [1] to [22], wherein Z is -CN group, -CO₂CH₃ group, or -OH group;
[24] an embodiment as in any one of [1] to [23], wherein a is 0;
[25] an embodiment as in any one of [1] to [24], wherein b is 1;
[26] an embodiment as in any one of [1] to [25], wherein c is 0;
[27] an embodiment as in any one of [1] to [26], wherein Y is -CH₂- group where d is no less than 1;
[28] an embodiment as in any one of [1] to [27], wherein d is 0;
[29] an embodiment as in any one of [1] to [28], wherein the compound represented by the above general formula (1) does not have an -O-O- bond;
[30] a lubricant oil composition including (A) a lubricating base oil and (B) a compound represented by the above general formula (1) as in any one of [1] to [29];
[31] an embodiment as in [30], wherein the content of the component (B) is 0.05 to 10 weight% to the total amount of the composition;
[32] an embodiment as in [31], wherein the content of the component (B) is no more than 5 weight% to the total amount of the composition;
[33] an embodiment as in [31] or [32], wherein the content of the component (B) is no less than 0.1 weight% to the total amount of the composition;
[34] an embodiment as in any one of [30] to [33], wherein the kinematic viscosity at 100°C of (A) the lubricating base oil is 3.0 to 25.0 mm²/s;
[35] an embodiment as in any one of [30] to [34], wherein the lubricant oil composition is for internal combustion engines and the kinematic viscosity at 100°C of (A) the lubricating base oil is 3.0 to 16.3 mm²/s;
[36] an embodiment as in any one of [30] to [34], wherein the composition is for automatic transmissions and the kinematic viscosity of (A) the lubricating base oil at 100°C is 3.5 to 25.0 mm²/s;
[37] an embodiment as in any one of [30] to [36], wherein the lubricant oil composition further includes one or more additive selected from a group consisting of: an ashless dispersant, an antioxidant, a friction modifier other than the compound represented by the general formula (1), an anti-wear agent, an extreme pressure agent, a metallic detergent, a viscosity index improver, a pour point depressant, a corrosion inhibitor, an anti-rust agent, a metal deactivator, a demulsifier, a deforming agent, and a coloring agent;
[38] an embodiment as in any one of [30] to [37], wherein the lubricant oil composition includes an ashless dispersant in an amount of 0.01 to 20 weight% on the basis of the total amount of the composition;
[39] an embodiment as in any one of [30] to [38], wherein the lubricant oil composition includes an antioxidant (excepting the compound represented by the general formula (1)) in an amount of 0.1 to 5.0 weight% on the basis of the total amount of the composition;
[40] an embodiment as in any one of [30] to [39], wherein the lubricant oil composition includes a friction modifier other than the compound represented by the general formula (1) in an amount of 0.05 to 5 weight% on the basis of the total amount of the composition;
[41] an embodiment as in any one of [30] to [40], wherein the lubricant oil composition includes an anti-wear agent or an extreme pressure agent (excepting the compound represented by the general formula (1)) in an amount of 0.05 to 5 weight% on the basis of the total amount of the composition;
[42] an embodiment as in any one of [30] to [41], wherein the lubricant oil composition includes a metallic detergent in an amount of 0.01 to 5 weight% as a metal content on the basis of the total amount of the lubricant oil composition;
[43] an embodiment as in [42], wherein the lubricant oil composition is used for lubrication of automatic transmissions or continuously variable transmissions;
[44] an embodiment as in any one of [30] to [42], wherein the lubricant oil composition includes a metallic detergent in an amount of 0.01 to 1.0 weight% as a metal content on the basis of the total amount of the lubricant oil composition; and
[45] an embodiment as in [44], wherein the lubricant oil composition is used for lubrication of internal combustion engines.

### Examples

Hereinafter the present invention will be further specifically described with reference to Examples and Comparative Examples. It is noted that the following Examples are intended as examples of the present invention, and not intended to limit the present invention.

### <Preparation example>

Biphenyl-based friction modifiers L5 and L6 according to the first aspect of the present invention and a friction modifier C2 outside the scope of the present invention were prepared.

### (Preparation example 1)

The biphenyl friction modifier L5 wherein in the general formula (1), R = decyl group, X¹ = -O- group, X² = single bond, Z = -CO₂CH₃ group, a = 0, b = 1, c = 0, d = 0 was prepared by the following procedures.

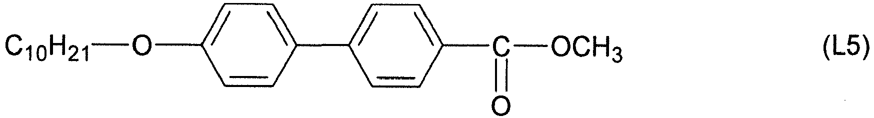

To a 100 mL three-necked flask equipped with a Dimroth condenser, 2.26 mmol (0.80 g) of 4'-decyloxybiphenylcarboxylic acid (manufactured by TOKYO CHEMICAL INDUSTRY CO., LTD.), 3.39 mmol (0.40 g) of thionyl chloride (manufactured by TOKYO CHEMICAL INDUSTRY CO., LTD.), 50 mL of toluene, and a catalytic amount of N,N-dimethylformamide were added, and the atmosphere inside the flask was substituted with nitrogen. The resultant mixture was stirred and heated to reflux toluene. After refluxing for three hours, toluene and unreacted thionyl chloride were removed by distillation. After the distillation, 50 mL of methanol and 10 mL of triethylamine were added and the atmosphere inside the flask was substituted with nitrogen. The resultant mixture was stirred and heated to reflux methanol. After refluxing for two hours, unreacted methanol and triethylamine were removed by distillation. After the distillation, the obtained solid was washed with water to remove triethylamine hydrochloride, and thereafter dried to afford 0.72 g of the target compound. The progress of the reaction was confirmed by a ¹H-NMR spectrum.

### (Preparation example 2)

The biphenyl friction modifier L6 wherein in the general formula (1), R = heptadecyl group, X¹ = -COO- group, X² = single bond, Z = -OH group, a = 0, b = 1, c = 0, d = 0, and a friction modifier C2 outside the scope of the present invention wherein in the above general formula (1), R = heptadecyl group, X¹ = -COO- group, X² = single bond, a = 0, b = 1, c = 0, d = 0, and X² - Z = -OCOC₁₇H₃₃ were prepared.

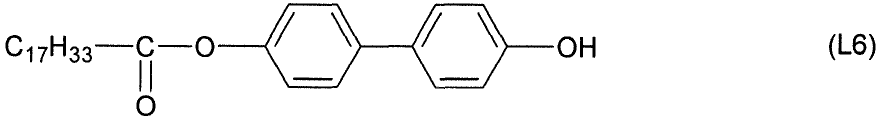

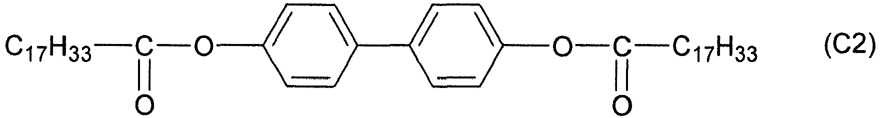

To a four-necked flask, 99.69 mmol (19.42 g) of 4,4'-biphenol (manufactured by TOKYO CHEMICAL INDUSTRY CO., LTD.), 50 mL of tetrahydrofuran, and 20 mL of triethylamine were added, and thereafter the atmosphere inside the flask was substituted with nitrogen. To the resultant mixture, 9.97 mmol (3.00 g) of an oleic acid chloride (manufactured by TOKYO CHEMICAL INDUSTRY CO., LTD.) and 50 mL of tetrahydrofuran in a dropping funnel were added dropwise for 40 minutes while stirring at a room temperature. Thereafter, triethylamine hydrochloride formed was removed by filtration, and the solvent of the filtrate was removed by a rotary evaporator. The obtained solid was washed with water to remove the remaining triethylamine hydrochloride. The washed sold was dried, and added to a 500 mL flask with 300 mL of toluene, and the resultant mixture was stirred to dissolve the solid. Unreacted 4,4'-biphenol, which was insoluble in toluene, was removed by filtration, and the solvent of the filtrate was removed by a rotary evaporator, whereby a mono/bis mixture of biphenyl ester was obtained. The obtained mono/bis mixture and 300 mL of methanol were added to a 500 mL flask and stirred, and only the mono-esterified product was dissolved. The bis-esterified product was separated by filtration and dried, whereby 2.20 g of bis(biphenyl) ester (above formula (C2)) was obtained. The solvent was removed from the filtrate by a rotary evaporator, and 1.37 g of the target compound (above formula (L6)) was obtained. The progress of the reaction was confirmed by a ¹H-NMR spectrum.

### <Examples 1 to 6 and Comparative Examples 1 to 5>

As shown in Table 1, lubricant oil compositions of the present invention (Examples 1 to 6), and lubricant oil compositions for comparison (Comparative Examples 1 to 5) were each prepared. In Table 1, the unit "wt.%" means weight%. Structural formulas of the friction modifiers L1 to L6 used in Examples 1 to 6, and the friction modifiers C1 to C4 used in Comparative Examples 1 to 4 are shown below.

**[Table 1]**

| | Example 1 | Example 2 | Example 3 | Example 4 | Example 5 | Example 6 | Comparative Example 1 | Comparative Example 2 | Comparative Example 3 | Comparative Example 4 | Comparative Example 5 |
|---|---|---|---|---|---|---|---|---|---|---|---|
| (A) base oil (*1) | balance | balance | balance | balance | balance | balance | balance | balance | balance | balance | balance |
| (B) friction modifier | | | | | | | | | | | |
| (B-1) L1 wt.% | 0.5 | - | - | - | - | - | - | - | - | - | - |
| (B-2) L2 wt.% | - | 0.5 | - | - | - | - | - | - | - | - | - |
| (B-3) L3 wt.% | - | - | 0.5 | - | - | - | - | - | - | - | - |
| (B-4) L4 wt.% | - | - | - | 0.5 | - | - | - | - | - | - | - |
| (B-5) L5 wt.% | - | - | - | - | 0.5 | - | - | - | - | - | - |
| (B-6) L6 wt.% | - | - | - | - | - | 0.5 | - | - | - | - | - |
| (B-7) C1 wt.% | - | - | - | - | - | - | 0.5 | - | - | - | - |
| (B-8) C2 wt.% | - | - | - | - | - | - | - | 0.5 | - | - | - |
| (B-9) C3 wt.% | - | - | - | - | - | - | - | - | 0.5 | - | - |
| (B-10) C4 wt.% | - | - | - | - | - | - | - | - | - | 0.5 | - |
| friction coefficient | 0.164 | 0.169 | 0.155 | 0.151 | 0.157 | 0.079 | 0.197 | 0.186 | 0.181 | 0.176 | 0.213 |

| | | | | | | | | | | | |
|---|---|---|---|---|---|---|---|---|---|---|---|
| 1) poly-α-olefin base oil, kinematic viscosity at 100°C: 1.7 mm²/s, kinematic viscosity at 40 °C: 5.0 mm²/s, pour point: -66°C | | | | | | | | | | | |

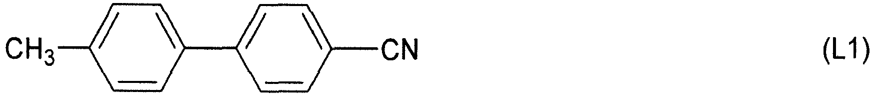

(L1 was purchased from Tokyo Chemical Industry Co., Ltd., and used as it was.) 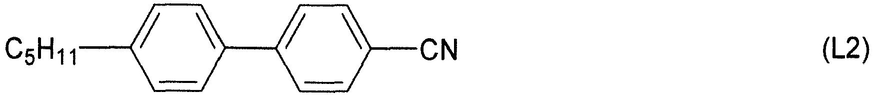 (L2 was purchased from Tokyo Chemical Industry Co., Ltd., and used as it was.) 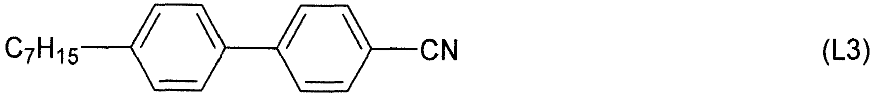 (L3 was purchased from Tokyo Chemical Industry Co., Ltd., and used as it was.) 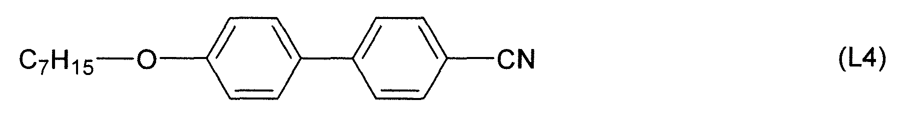 (L4 was purchased from Tokyo Chemical Industry Co., Ltd., and used as it was.) 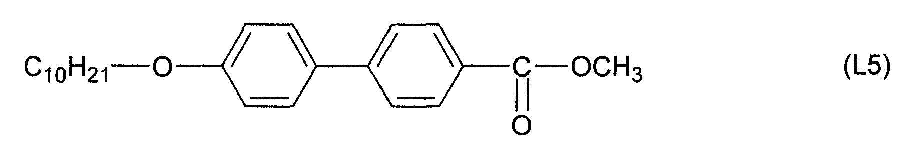 (L5 was prepared in the preparation example 1.) 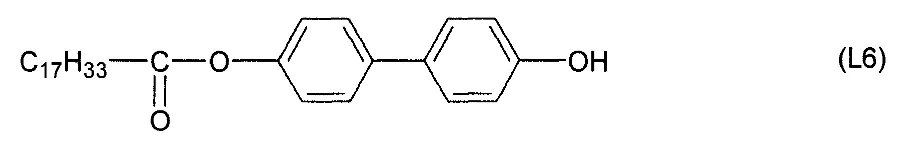 (L6 was prepared in the preparation example 2.) 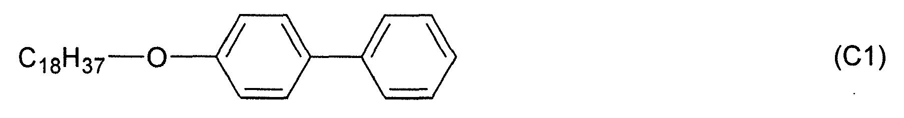 (C1 was purchased from Tokyo Chemical Industry Co., Ltd., and used as it was.) 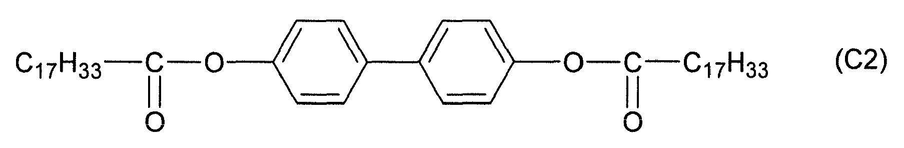 (C2 was prepared in the preparation example 2.) 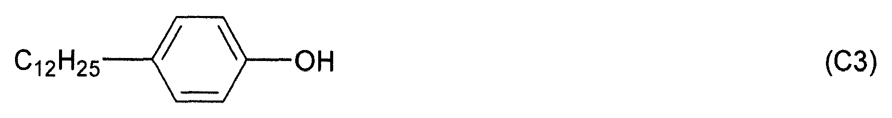 (C3 was purchased from KANTO CHEMICAL CO., INC., and used as it was.) 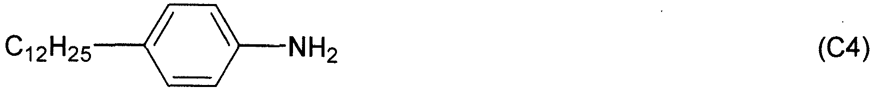 (C4 was purchased from CHUO KASEIHIN CO., INC., and used as it was.)

### (Evaluation method)

The friction characteristic of each of the prepared lubricant oil compositions was evaluated. The friction characteristic was evaluated by: measuring the friction coefficient by means of a cylinder-on-desk reciprocating sliding tester (SRV manufactured by Optimol), under conditions of 700 N of load, 0.4 GPa of surface pressure (maximum Hertz stress), 10 Hz of frequency, 1 mm of amplitude, 100°C of temperature, 60 minutes of testing time. The friction characteristic was evaluated by calculating the average friction coefficient which is an averaged friction coefficient for the time of 30 to 60 minutes after beginning of the test. This measurement conditions correspond to the conditions of boundary lubrication. Fig. 1 shows a graph in which the measured friction coefficient of each lubricant oil composition is plotted.

Fig. 1 is a graph to compare the test results of Examples 1 to 6 and Comparative Examples 1 to 5. The lubricant oil of Comparative Example 5 consists of a base oil only. The friction reducing effect of each lubricant oil composition is evaluated by the comparison with the friction coefficient of the lubricant oil of Comparative Example 5.

As shown in Fig. 1, the lubricant oil compositions of Examples 1 to 6 showed good friction reducing effects. Among them, the lubricant oil compositions of Example 3 to 6 which respectively include L3 to L6 wherein in the general formula (1), R has a carbon number of no less than 6, showed better friction reducing effects. Particularly, the lubricant oil composition of Example 6 including L6 showed especially superior friction reducing effect.

The lubricant oil compositions of Comparative Example 1 and 2 which respectively include C1 and C2 not having Z in the general formula (1), and the lubricant oil compositions of Comparative Example 3 and 4 which respectively include C3 and C4 not having a biphenyl moiety were inferior to the lubricant oil compositions of Examples 1 to 6 in friction reducing effect.

From the above test results, it was shown that it was possible to provide an ashless oiliness agent friction modifier having a good friction reducing effect, according to the lubricant additive of the present invention.

### Industrial Applicability

The lubricant additive and lubricant oil composition of the present invention may be preferably used for lubrication of various kinds of machines, and especially preferably used for lubrication of internal combustion engines and transmissions.

## Claims

1. A lubricant additive comprising:
a compound represented by a general formula (1): 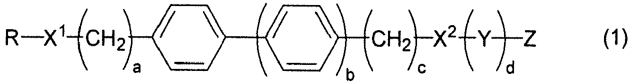
(In the general formula (1), R is a hydrocarbon-containing group having a carbon number of no less than 1;
X¹ is a single bond, -CONH- group, -NHCO- group, -NH- group, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
X² is a single bond, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
Z is -CN group, -CO₂CH₃ group, or a group having a carbon number of 0 to 6 and having no less than one heteroatom and a hydrogen atom bonded to the heteroatom, wherein the heteroatom is selected from a group consisting of: oxygen, nitrogen, sulfur, and phosphorus;
a is 0 or 1;
b is 1 or 2;
c is 0 or 1;
d is an integer of 0 to 2; and
Y is -CH₂- group or -OCH₂CH₂- group where d is no less than 1.).

2. A lubricant oil composition comprising:
(A) a lubricating base oil; and
(B) a compound represented by a general formula (1): 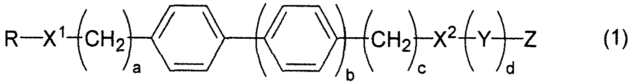
(In the general formula (1), R is a hydrocarbon-containing group having a carbon number of no less than 1;
X¹ is a single bond, -CONH- group, -NHCO- group, -NH- group, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
X² is a single bond, or a linking group having a carbon number of 0 to 1 and having no less than one heteroatom and not having a hydrogen atom bonded to the heteroatom, wherein the heteroatom is selected from a group consisting of: oxygen, nitrogen, and sulfur;
Z is -CN group, -CO₂CH₃ group, or a group having a carbon number of 0 to 6 and having no less than one heteroatom and a hydrogen atom bonded to the heteroatom, wherein the heteroatom is selected from a group consisting of: oxygen, nitrogen, sulfur, and phosphorus;
a is 0 or 1;
b is 1 or 2;
c is 0 or 1;
d is an integer of 0 to 2; and
Y is -CH₂- group or -OCH₂CH₂- group where d is no less than 1.).

3. The lubricant oil composition according to claim 2, further comprising:
one or more additive selected from a group consisting of: an ashless dispersant, an antioxidant, a friction modifier other than the compound represented by the general formula (1), an anti-wear agent, an extreme pressure agent, a metallic detergent, a viscosity index improver, a pour point depressant, a corrosion inhibitor, an anti-rust agent, a metal deactivator, a demulsifier, a deforming agent, and a coloring agent.
